# EUROPÄISCHE PATENTSCHRIFT

(11) **EP 2 210 642 B1**
(45) Veröffentlichungstag und Bekanntmachung des Hinweises auf die Patenterteilung: **30.03.2016**
(21) Anmeldenummer: 10000598.2
(22) Anmeldetag: 21.01.2010
(51) Int. Cl.: A61N 2/02

(54) **Magnetfeldapplikator**
Magnetic field applicator
Applicateur de champ magnétique

(30) Priorität: 26.01.2009 DE 102009006165
(43) Veröffentlichungstag der Anmeldung: 28.07.2010
(73) Patentinhaber: Kafka, Wolf A., Prof. Dr., 82288 Kottgeisering (DE)
(72) Erfinder: Kafka, Wolf A., Prof. Dr., 82288 Kottgeisering (DE)
(74) Vertreter: Epping - Hermann - Fischer

(56) Entgegenhaltungen:
- DE-A1- 4 004 682
- DE-A1- 19 615 647

## Beschreibung

Die Erfindung betrifft einen Magnetfeldapplikator mit mehreren zur Erzeugung eines Magnetfelds geeigneten elektrischen Leitern.

Magnetfeldapplikatoren werden in verschiedenen Ausführungsformen in Form von Matten hergestellt, in alternativen Ausführungsformen sind Magnetfeldapplikatoren mit einem Gehäuse ausgeführt. Magnetfeldapplikatoren dienen beispielsweise der nicht-invasiven therapeutischen Behandlung von lebendem Gewebe. Hierbei ist sowohl die Behandlung von menschlichen Körpern, aber auch von Tieren möglich.

Magnetfeldapplikatoren in Form von Matten eignen sich insbesondere für die Ganzkörperbehandlung am Menschen. Für Teilkörperbehandlungen können unter anderem die übrigen genannten Ausführungsformen verwendet werden.

Die verschiedenen Magnetfeldapplikatoren weisen üblicherweise elektrische Leiter auf, welche an ein Steuergerät angeschlossen werden. Dieses gibt einen zeitlich veränderlichen elektrischen Strom bzw. eine entsprechende Spannung an die elektrischen Leiter ab, um dadurch ein sich zeitlich veränderndes Magnetfeld zu erzeugen, welches vom Magnetfeldapplikator abgegeben wird. Wenn das so erzeugte Magnetfeld auf lebendes Gewebe wirkt, ergibt sich in Abhängigkeit des zeitlichen Magnetfeldintensitätsverlaufs die beabsichtigte therapeutische Wirkung im Gewebe. Diese gewünschte Wirkung wird auch als Elektro-Magnetfeldbehandlung bezeichnet.

Ein mattenförmiger Magnetfeldapplikator ist beispielsweise aus der DE 103 04 093 A1 bekannt. In der dort beschriebenen Matte weist der Magnetfeldapplikator mehrere kreisförmige Leiterbahnen auf, die über die Fläche der Matte verteilt sind.

Eine Matte für Ganzkörperbehandlung ist jedoch nicht für die Behandlung kleinerer Körperregionen geeignet, da bei herkömmlichen Matten das erzeugte Magnetfeld mit auf die ganze Mattenfläche verteilter Intensität abgegeben wird. Aus diesem Grund werden vielfach kleinere Matten eingesetzt, die in ihrer Größe für eine Teilkörperbehandlung eingerichtet sind. Derartige kleinere Matten können zwar unter Umständen mit dem gleichen Steuergerät betrieben werden, jedoch ist es notwendig, für verschiedene Anwendungsfälle kleinere Matten verschiedener Größe bereitzustellen. Dadurch, dass für verschiedene Behandlungsformen unterschiedlich große Magnetfeldapplikatoren notwendig sein können, entstehen sowohl in der Produktion als auch für den Endverbraucher hohe Kosten.

Magnetfeldapplikatoren werden zu Transport und Aufbewahrung üblicherweise ohne spezielle Vorgaben zusammengerollt oder beliebig gefaltet und beispielsweise zusammen mit entsprechenden Steuergeräten und/oder weiteren Applikatorformen in entsprechend dimensionierte Behältnisse verpackt. Beim Zusammenrollen oder beliebigen Falten durch den Anwender kann es jedoch zu Beschädigungen oder Veränderungen der Leiterbahnen kommen, so dass die gewünschte Magnetfelderzeugung verändert oder sogar unmöglich wird.

Das Dokument DE 4004682 A1 zeigt eine Magnetdecke mit mehreren verteilt angeordneten Induktionsspulen. Die Magnetdecke lässt sich zum Transport in vier Längsabschnitten falten.

Das Dokument DE 19615647 A1 zeigt eine nicht faltbare Spulenmatte mit mehreren überlappend angeordneten Induktionsspulen, um eine gleichmäßige Feldverteilung über die Matte zu erreichen.

Eine zu lösende Aufgabe besteht demnach darin, einen Magnetfeldapplikator bereitzustellen, der sich in seiner Anwendung durch verringerten Aufwand und erhöhte Flexibilität auszeichnet.

Diese Aufgabe wird durch den Gegenstand des unabhängigen Patentanspruchs gelöst. Weiterbildungen und verschiedene Ausführungsformen sind in den abhängigen Patentansprüchen gekennzeichnet.

Ein Ausführungsbeispiel eines Magnetfeldapplikators umfasst einen flächigen Träger, der durch wenigstens zwei sich schneidende mechanisch definierte Faltungslinien in eine Anzahl Felder geteilt bzw. unterteilt und entlang der Faltungslinien faltbar ist. Hierbei weisen wenigstens zwei Felder aus der Anzahl von Feldern eine Magnetfeldquelle auf, die jeweils einen zur Erzeugung eines Magnetfelds geeigneten elektrischen Leiter umfasst.

Der flächige Träger, der beispielsweise als Matte ausgeführt ist, lässt sich entlang der mechanisch definierten Faltungslinien so falten, dass die durch die Faltungslinien gebildeten Felder in verschiedenen Formen oder Ausgestaltungen übereinander zu liegen kommen. Beispielsweise weist der Träger bzw. die Matte eine Oberund eine Unterschicht auf, zwischen denen die Magnetfeldquellen angeordnet sind. Durch die Faltungslinien lässt sich beispielsweise ein flächiger Träger in Körpergröße durch die Faltung gezielt und definiert verkleinern. Da die Felder nicht in sich sondern lediglich entlang der Faltungslinien gefaltet werden, wird eine Beschädigung der elektrischen Leiter zur Magnetfelderzeugung in den jeweiligen Feldern verhindert.

Wenn bei einem Magnetfeldapplikator in gefaltetem Zustand mehrere Felder mit entsprechenden elektrischen Leitern übereinander liegen, wirken im Betrieb des Magnetfeldapplikators die von den übereinander liegenden Feldern erzeugten Magnetfelder zusammen. Dies kann bei entsprechender Ansteuerung der Felder ausgenutzt werden, wenn der Magnetfeldapplikator in gefaltetem Zustand betrieben wird. Ein solcher Magnetfeldapplikator ist somit flexibler einsetzbar.

Die Magnetfeldquellen sind derart in den Feldern angeordnet, dass sich Magnetfeldintensitäten der Magnetfeldquellen übereinander gefalteter Felder bei einer Faltung des Trägers erhöhend überlagern. Wenn beispielsweise zwei benachbarte Felder gleiche Magnetfeldintensität erzeugen, kann sich die Magnetfeldintensität bei einer Faltung dieser Felder und entsprechender Polarität der der einzelnen Magnetfeld erzeugenden Leiter näherungsweise verdoppeln. Werden weitere Felder gleicher Intensität bei richtiger Polarität übereinander gefaltet, kann es auch zu einer Verdreifachung bzw. Vervierfachung usw. kommen. Alternativ kann auch die Intensität des elektrischen Stroms bzw. der elektrischen Spannung, welche zur Ansteuerung der elektrischen Leiter verwendet werden, in entsprechender Weise reduziert werden, um bei Übereinanderfaltung der Felder eine gewünschte Intensität zu erreichen. Somit lässt sich ein und derselbe Magnetfeldapplikator für verschiedene Anwendungen einsetzen, z.B. zur Ganzkörperbehandlung oder zur Behandlung von einzelnen Körperteilen, wobei der Einsatz von der Ansteuerung und der so erzeugten Intensität abhängen kann.

In einer Ausführungsform sind die Faltungslinien durch Nähte auf dem Träger gebildet. Somit stellen diese Nähte die mechanische Definition der Faltungslinien dar. Durch die Nähte ist vorzugsweise eine Dicke des flächigen Trägers an den Faltungslinien reduziert, so dass sich eine entsprechende Faltung leichter bewerkstelligen lässt.

Alternativ oder zusätzlich können benachbarte Felder durch Scharniere oder Bänder miteinander verbunden sein, deren Achsen die Faltungslinien definieren. Hierbei soll unter Scharnier jedes mechanische Element verstanden werden, welches einen Drehpunkt um eine Achse des Elements ermöglicht. In ähnlicher Weise ist ein Band zu sehen, welches sich vom Scharnier im Wesentlichen dadurch unterscheidet, dass hier auf den jeweiligen Feldern angeordneten Glieder des Bandes trennbar sind. Vorzugsweise ist ein Drehwinkel das Scharniers oder Bandes im Wesentlichen unbegrenzt, so dass durch das Scharnier oder Band eine Faltung nicht auf eine Faltungsrichtung begrenzt ist.

In einer Ausführungsform weisen benachbarte Felder Platten auf, die entlang einer der Faltungslinien flexibel miteinander verbunden sind. Anders ausgedrückt sind die Platten im Wesentlichen starr, während eine Faltung entlang der flexiblen Verbindung möglich ist. Die Platten sind hierbei beispielsweise biegsam, jedoch vorzugsweise nicht faltbar.

In einem Ausführungsbeispiel sind die Faltungslinien sichtbar. Somit kann ein Anwender des Magnetfeldapplikators, der diesen falten will, sofort sehen, wie und wo er den flächigen Träger falten kann.

In verschiedenen Ausführungsformen sind die elektrischen Leiter als flache Spulen ausgeführt. Beispielsweise sind die elektrischen Leiter mittels eines Druck- oder Ätzverfahrens auf einer Folie aufgebracht. Alternativ oder zusätzlich kann zumindest einer der elektrischen Leiter über Nähte in den Feldern fixiert sein. Dadurch ist bei einer Faltung des Trägers zusätzlich gewährleistet, dass der elektrische Leiter bzw. die magnetfelderzeugende Spule nicht verrutschen und die Eigenschaften des Magnetfelds nicht verändert werden.

Die Spulen können in verschiedenen Kombinationen zwischen einem ersten und einem zweiten Anschluss in Reihe und/oder parallel geschaltet sein. Beispielsweise ist eine Serienschaltung einer Hälfte von entsprechenden Spulen parallel zu einer Serienschaltung der anderen Hälfte entsprechender Spulen geschaltet. Jedoch können auch andere Kombinationen von Serien- und Parallelschaltungen verwendet werden, wobei die jeweils gewählte Kombination bei der Zuführung eines entsprechenden Steuersignals an den ersten und zweiten Anschluss zu berücksichtigen ist, insbesondere in einer absoluten Höhe des zugeführten Stroms bzw. der angelegten Spannung.

In einem weiteren Ausführungsbeispiel sind die elektrischen Leiter zusätzlich zur Wärmeerzeugung geeignet. Hierbei wird einer der elektrischen Leiter vorzugsweise so ausgeführt, dass sein elektrischer Widerstand zumindest einen Teil der Energie, die dem elektrischen Leiter als Strom oder Spannung zugeführt wird, in Wärme umsetzt. Durch die erzeugte Wärme können die Effekte des weiterhin vorhandenen elektromagnetischen Felds auf das lebende Gewebe unterstützt werden.

In einem weiteren Ausführungsbeispiel weist wenigstens eine der Magnetfeldquellen zusätzlich einen Permanentmagneten auf. Dadurch lässt sich ein magnetisches Grundfeld statischer Art erzeugen, welches zusammen mit dem im Betrieb des Magnetfeldapplikators erzeugten elektromagnetischen Wechselfeld wirkt. Vorzugsweise sind, wenn mehrere der Felder Permanentmagneten aufweisen, diese so angeordnet, dass bei einer Faltung des flächigen Trägeres eine erhöhende Überlagerung der jeweiligen statischen Magnetfelder erzielt wird. Anders ausgerückt sind in diesem Fall die Permanentmagneten so angeordnet, dass sich ihre Magnetfelder nicht gegenseitig auslöschen.

Die Felder des Magnetfeldapplikators bzw. des flächigen Trägers sind in verschiedenen Ausführungsformen derart ausgestaltet, dass sie im Wesentlichen die gleichen Abmessungen aufweisen. In diesem Fall kann bei Übereinanderfaltung aller Felder eine einheitliche Form des gefalteten Träger erreicht werden.

Die Magnetfeldquellen der einzelnen Felder können so gestaltet sein, dass durch sie im Betrieb unterschiedlich große Magnetfeldintensitäten erzeugt werden. Vorzugsweise sind die Magnetfeldquellen bzw. deren elektrische Leiter so dimensioniert und angeordnet, dass im Betrieb von ihnen die gleiche Magnetfeldintensität ausgeht. Beim Übereinanderfalten mit entsprechend vorgesehener Polarität der Magnetfeldquellen kommt es so im Wesentlichen zu einer ganzzahligen Vervielfachung der Magnetfeldintensität einer einzelnen Magnetfeldquelle.

In den beschriebenen Ausführungsbeispielen für den Magnetfeldapplikator kann das Material des Trägers beispielsweise einen textilen Stoff, ein Vlies, ein Gewebe, eine Membran, eine Kunststofffolie oder eine beliebige Kombination der genannten Materialien umfassen.

Nachfolgend wird die Erfindung in Ausführungsbeispielen unter Zuhilfenahme der Zeichnungen beschrieben. Gleiche oder gleichartige Elemente sind hierbei mit gleichen Bezugszeichen gekennzeichnet.

Es zeigen:
- Figur 1: ein Ausführungsbeipiel eines Magnetfeldapplikators,
- Figur 2: eine beispielhafte Detailansicht des Magnetfeldapplikators,
- Figur 3: Ausführungsbeispiele für die Verbindung zweier Felder,
- Figur 4: ein Ausführungsbeispiel eines spulenförmigen elektrischen Leiters,
- Figur 5: eine beispielhafte Detailansicht eines spulenförmigen elektrischen Leiters,
- Figur 6: ein Ausführungsbeispiel eines Feldes mit Magnetfeldquellen,
- Figur 7: eine beispielhafte schematische Anordnung für die Verschaltung mehrerer elektrischer Leiter
- Figur 8: ein Ausführungsbeispiel einer Verschaltung von als Spulen ausgeführter elektrischer Leiter, und
- Figur 9: ein Ausführungsbeispiel für einen Faltungsvorgang eines beispielhaften Magnetfeldapplikators.

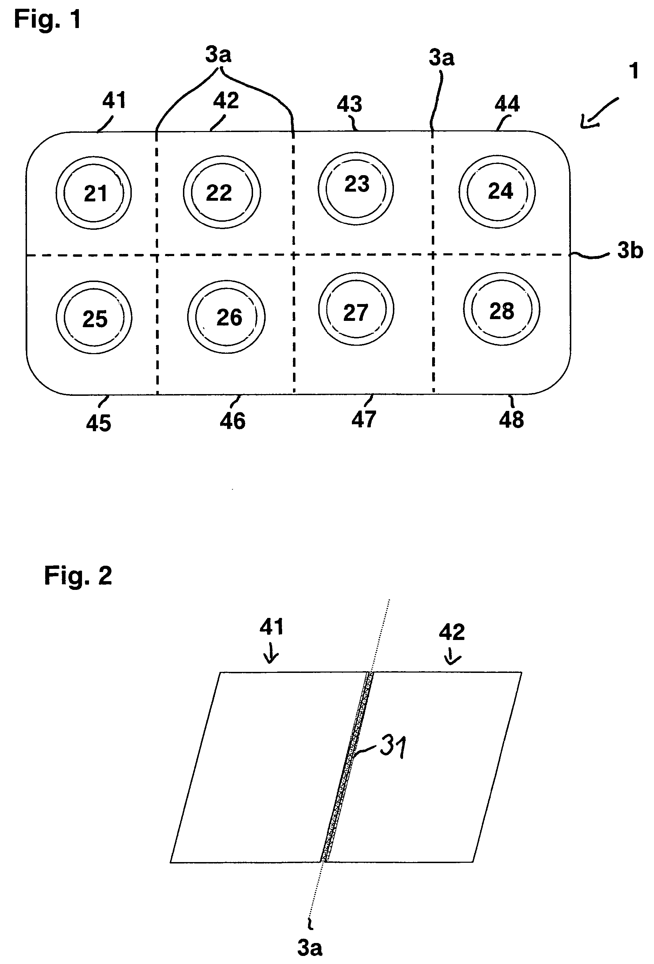

Figur 1 zeigt ein Ausführungsbeispiel eines Magnetfeldapplikators mit einem flächigen Träger 1. Der Träger 1 weist in diesem Ausführungsbeispiel acht Felder 41 bis 48 auf, die durch Faltungslinien 3a, 3b voneinander getrennt sind. Anders ausgedrückt ist der flächige Träger 1 durch die Faltungslinien 3a, 3b in die Felder 41 bis 48 aufgeteilt. Jedes der Felder weist eine Magnetfeldquelle 21 bis 28 auf, welche einen hier ringförmig angedeuteten Leiter umfasst.

Die Zahl und Anordnung der Felder kann auch variiert werden. Beispielsweise kann der flächige Träger 1 mehr als eine horizontale Faltungslinie 3b aufweisen. Ebenso ist es möglich, dass die im Ausführungsbeispiel dargestellte Zahl von 3 vertikalen Faltungslinien 3a verändert wird.

Die Faltungslinien 3a, 3b sind mechanisch im oder auf dem flächigen Träger 1 definiert. Durch die mechanische Definition der Faltungslinien 3a, 3b lässt sich der Träger 1 gezielt falten, so dass es nicht zu einer Beschädigung der Magnetfeldquellen 21 bis 28 in den Feldern 41 bis 48 kommen kann. Eine derartige Faltung kann sowohl für die Aufbewahrung bzw. den Transport des Magnetfeldapplikators sinnvoll sein, als auch für den Betrieb des Magnetfeldapplikators.

Im Betrieb kann der Magnetfeldapplikator nämlich sowohl im ungefalteten als auch im gefalteten Zustand verwendet werden. Bei Versorgung der elektrischen Leiter in den Magnetfeldquellen 21 bis 28 mit einem entsprechenden elektrischen Steuersignal wird von jeder der Magnetquellen ein Magnetfeld mit entsprechend dem Steuersignal variierender Magnetfeldintensität abgegeben. In ungefaltetem Zustand ergibt sich somit eine Abgabe des Magnetfelds über die Fläche des Trägers 1 verteilt. Im gefalteten Zustand des Trägers 1 können die einzelnen Magnetquellen 21 bis 28 in den Feldern 41 bis 48 derart ausgerichtet sein bzw. angesteuert werden, dass sich die Magnetfeldintensitäten übereinander liegender Felder verstärken. Hierbei ist zu berücksichtigen, dass die Magnetfeldquellen bei angenommener gleicher Magnetfeldintensität so wirken, dass sich das elektromagnetische Feld nicht gegenseitig auslöscht sondern erhöhend überlagert. Somit kann bei gleich bleibendem Steuersignal der Magnetfeldquellen durch Faltung des Trägers 1 eine entsprechend erhöhte Magnetfeldintensität erreicht werden. Alternativ kann zum Erreichen einer, nach Faltung, gleich bleibenden Magnetfeldintensität auf kleinerer Fläche das Steuersignal entsprechend angepasst, also beispielsweise in seiner Intensität verringert werden. Beispielsweise wird die Intensität um einen Faktor reduziert, der der Anzahl übereinander gefalteter Magnetfeldquellen entspricht.

Figur 2 zeigt ein Ausführungsbeispiel für die mechanische Definition einer Faltungslinie, ausschnittweise dargestellt mit Feldern 41, 42 des hier nicht vollständig dargestellten Trägers 1. In diesem Ausführungsbeispiel ist die Faltungslinie 3a durch entsprechende Nähte 31 zwischen den Feldern 41, 42 gebildet. Durch die Nähte 31 wird auch vorzugsweise eine Dicke des Trägers 1 entlang der Faltungslinie 3a reduziert, was zu einem vereinfachten und verbesserten Faltverhalten des Trägers 1 führt. Zudem ist durch die Naht die Faltungslinie für einen Benutzer des Magnetfeldapplikators sichtbar.

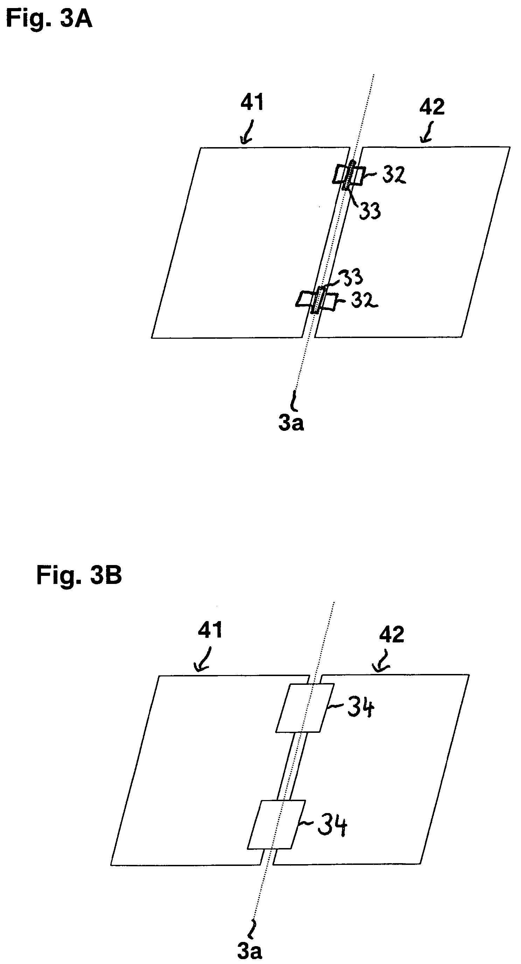

Zusätzlich oder alternativ können zu den Nähten 31 auch mechanische Elemente vorgesehen werden, welche eine Faltung des Trägers 1 entlang der Faltungslinie ermöglichen. Beispielsweise ist in Figur 3A als Ausführungsform eines derartigen mechanischen Elements die Verbindung zweier als Platten ausgeführter Felder 41, 42 als Scharnier 32 ausgeführt, welches um jeweilige Achsen 33 des Scharniers 32 beweglich ist. Somit ist die Faltungslinie 3a durch die Achsen 33 der Scharniere 32 definiert. Um eine möglichst flexible Faltung des Magnetfeldapplikators zu ermöglichen, ist es wünschenswert, dass die Scharniere 32 einen möglichst großen Drehwinkel aufweisen, vorzugsweise von nahezu 360°, um ein Aufeinanderfalten der Felder 41, 42 in beiden Richtungen zu ermöglichen. Wenn Scharniere 32 zur Verbindung mehrerer bzw. aller Felder des flächigen Trägers 1 verwendet werden, ist es günstig, die Scharniere derart anzuordnen, dass diese beim Übereinanderfalten der Felder möglichst nicht aufeinander zu liegen kommen. Somit kann eine möglichst geringe Faltungshöhe des Trägers 1 erreicht werden. Alternativ zu den Scharnieren 32 können auch Bänder verwendet werden, so dass eine Verbindung der Elemente gelöst werden könnte. Im Übrigen sollten die Bänder gleiche Funktion wie die zuvor beschriebenen Scharniere aufweisen.

Figur 3B zeigt ein weiteres Ausführungsbeispiel, bei dem Felder 41, 42 über Klebestreifen 34 flexibel miteinander verbunden sind. Beispielsweise kann der Klebestreifen 34 als Faserklebeband ausgeführt sein, um eine ausreichend hohe Stabilität zu erreichen.

In den Ausführungsbeispielen in den Figuren 3a und 3b weisen die Felder 41, 42 beispielsweise starre oder flexible Platten auf, welche mit den dargestellten Mitteln für die Faltung verbunden sind.

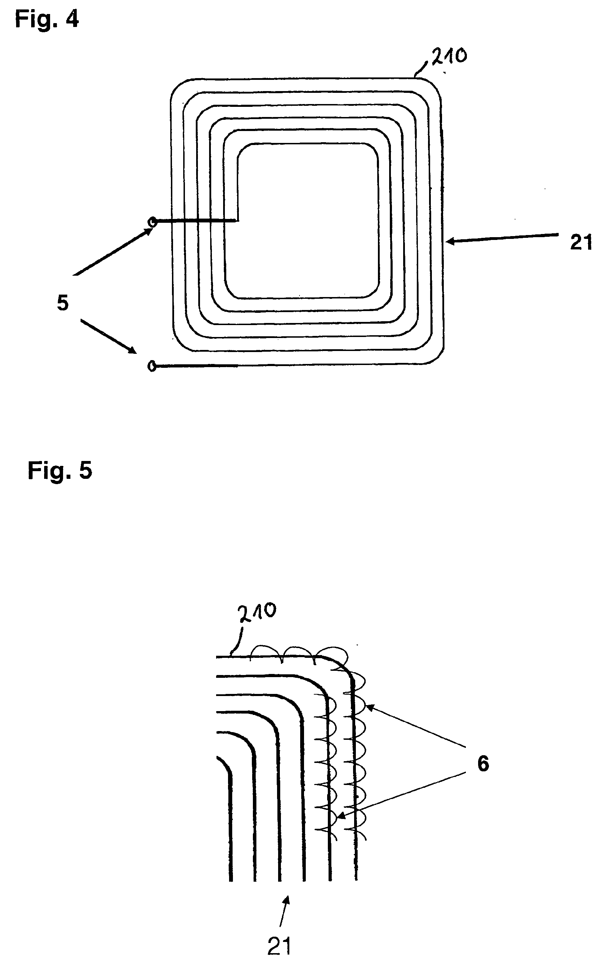

Figur 4 zeigt ein Ausführungsbeispiel für eine Magnetfeldquelle 21, welche jedoch auch für die übrigen Magnetfeldquellen 22 bis 28 eingesetzt werden kann. Die Magnetfeldquelle 21 ist hierbei als flache Spule 210 ausgeführt, bei der ein elektrischer Leiter spiralförmig angeordnet ist. Das äußere und das innere Ende der flachen Spule 210 sind an Anschlüsse 5 zur Zuführung eines elektrischen Signals angeschlossen. Die Spule 210 kann auf einem flachen Trägermaterial aufgebracht sein, welches wiederum auf dem flächigen Träger 1 in einem der Felder, hier das Feld 21, angeordnet wird. Beispielsweise ist der elektrische Leiter mittels eines Druck- oder Ätzverfahrens auf einer Folie aufgebracht. Wenn, wie zuvor bei den Figuren 3A und 3B beschrieben, die Felder starre oder flexible Platten aufweisen, kann die Spule 210 mit oder ohne Folie auf der Platte aufgebracht werden.

Figur 5 zeigt ein Detail einer Magnetfeldquelle 21, bei der der elektrische Leiter der Spule 210 über Nähte 6 fixiert ist. Beispielsweise ist der elektrische Leiter direkt im entsprechenden Feld vernäht. Dadurch kann ein Verrutschen des Leiters bzw. der Spule 210 im Feld verhindert werden.

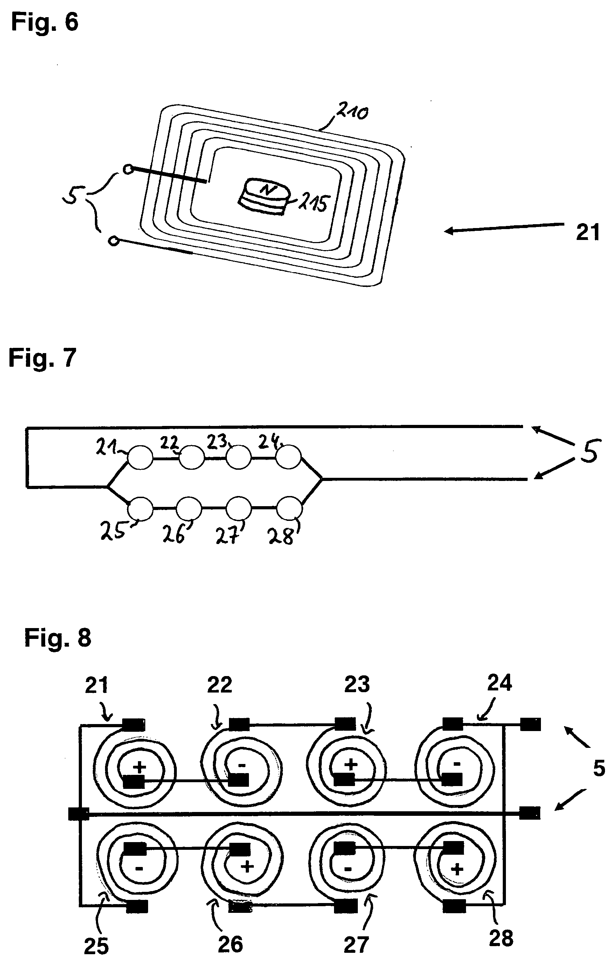

Figur 6 zeigt eine Abwandlung der Magnetfeldquelle 21 aus Figur 4. Zusätzlich zur Spule 210 ist in diesem Ausführungsbeispiel ein Permanentmagnet 215 vorgesehen, der beispielsweise mittig in der Spule 210 angeordnet ist. Durch die Permanentmagneten 215 wird ein statisches Magnetfeld erzeugt, das im Betrieb des Magnetfeldapplikators zusätzlich zu dem von der Spule 210 erzeugten Magnetfeld wirkt. Alternativ kann eines der Felder auch lediglich einen Permanentmagneten umfassen, also ohne, dass eine Spule oder ein Leiter vorhanden wäre.

Zusätzlich zur Magnetfelderzeugung können die in den Figuren 4, 5 und 6 gezeigten Spulen bei entsprechender Dimensionierung des elektrischen Leiters auch zur Wärmeerzeugung verwendet werden. Um die Spulen vor Beschädigung zu schützen, sind diese in den Feldern üblicherweise zwischen einer Oberschicht und einer Unterschicht des flächigen Trägers 1 eingebettet. Entsprechende Faltungslinien sind hierbei vorzugsweise auf Oberschicht und/oder Unterschicht sichtbar.

In Figur 7 ist eine schematische Verschaltung von Magnetfeldquellen 21 bis 28 zwischen einem ersten und einem zweiten Anschluss 5 dargestellt. Hierbei sind die Magnetfeldquellen 21, 22, 23, 24 in Reihe geschaltet. Ebenso bilden die Magnetfeldquellen 25, 26, 27, 28 eine entsprechende Reihenschaltung. Die beiden Reihenschaltungen sind zueinander parallel zwischen die Anschlüsse 5 geschaltet. Somit können die Magnetfeldquellen 21 bis 28 gemeinsam durch Zuführung eines einzigen elektrischen Signals an den Anschlüssen 5 angesteuert werden.

Figur 8 zeigt eine schematische Verschaltung der Magnetfeldquellen 21 bis 28, bei der eine entsprechende Polarität der Magnetfeldquellen berücksichtigt ist. Da im Betrieb des Magnetfeldapplikators im Wesentlichen Wechselsignale auf die Anschlüsse 5 gegeben werden, sind die mit einem Pluszeichen und einem Minuszeichen gekennzeichneten Polaritäten dahingehend zu verstehen, dass eine jeweils umgekehrte Polarität bewirkt wird. Dies ist aus dem Windungssinn des jeweiligen Anschluss der Magnetfeldquellen 21 bis 28 ersichtlich.

Beispielsweise wird die umgekehrte Polarität dadurch erreicht, dass jeweils innere Anschlüsse bzw. jeweils äußere Anschlüsse benachbarter Magnetfeldquellen miteinander verbunden sind. Im Speziellen sind das die inneren Anschlüsse der Magnetfeldquellen 21 und 22, 23 und 24, 25 und 26 sowie 27 und 28. Die Verschaltung der Magnetfeldquellen 21 bis 28 bezüglich Serien- und Parallelschaltungen entspricht der in Figur 7 dargestellten Anordnung. Die Verschaltung könnte aber beliebig variiert werden.

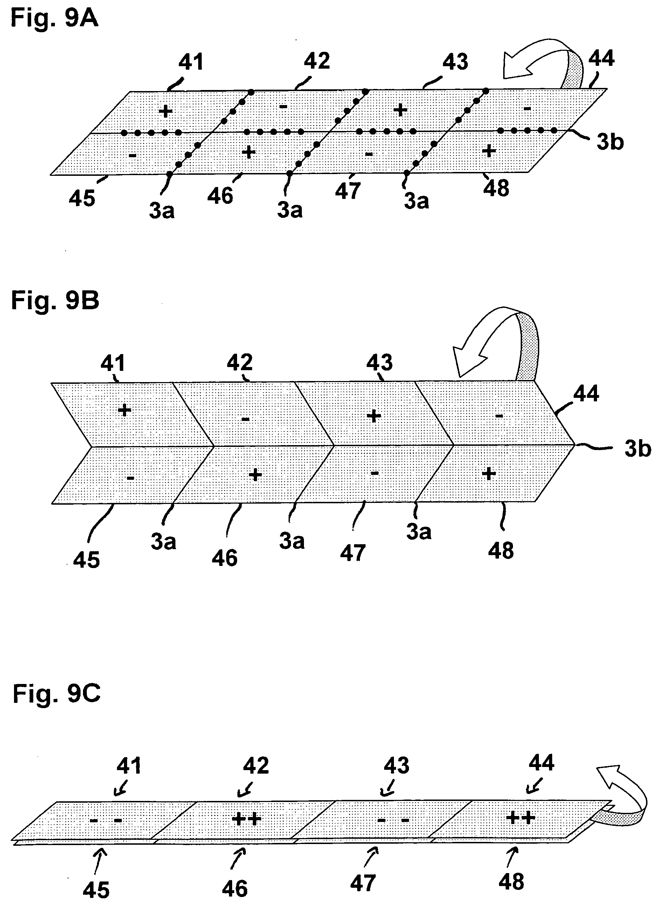

In den Figuren 9A bis 9E ist ein Faltungsvorgang des flächigen Trägers 1 mit entsprechend resultierenden Magnetfeldern dargestellt. Figur 9A zeigt einen zunächst ausgefalteten Träger mit Feldern 41 bis 48 und Faltungslinien 3a, 3b. Mit Verweis auf die Ausführungen aus Figur 8 sind in den Feldern 41, 43, 46, 48 Magnetfelder positiver Polarität im Betrieb des Magnetfeldapplikators dargestellt. Entsprechend weisen die Felder 42, 44, 45, 47 eine negative Polarität des Magnetfelds auf, also umgekehrt zu den jeweils benachbarten Feldern. Die Polarität der Magnetfelder ist zudem in der Draufsicht des Trägers 1 angenommen. In einer Ansicht von unten wären jeweils alle Polaritäten vertauscht. Ein erster Faltungsvorgang ist entlang der Faltungslinie 3b durch den gebogenen Pfeil angedeutet.

In Figur 9B ist ein teilweise gefalteter Träger 1 dargestellt. Hierbei werden die Felder 41 und 45, 42 und 46, 43 und 47 sowie 44 und 48 übereinander gefaltet.

In Figur 9C ist das Ergebnis dieser ersten Faltung dargestellt. Durch das Übereinanderfalten der Felder 41, 45 wirkt beispielsweise von oben gesehen weiterhin die negative Polarität des Feldes 45, während durch die Faltung die ebenfalls negative Polarität des Feldes 41, die aus der Untersicht herrührt, hinzukommt. Somit kommt es bei den übereinander gefalteten Feldern 41, 45 in der Draufsicht zu einer verdoppelten negativen Polarität, wenn ursprünglich gleiche Magnetfeldintensitäten angenommen werden.

In ähnlicher Weise überlagern sich die Magnetfeldintensitäten der Felder 42 und 46 zu einer verdoppelten positiven Polarität, ebenso wie bei den Feldern 44 und 48. Bei den Feldern 43 und 47 kommt es wie bei den Feldern 41 und 45 zu einer verdoppelten negativen Polarität.

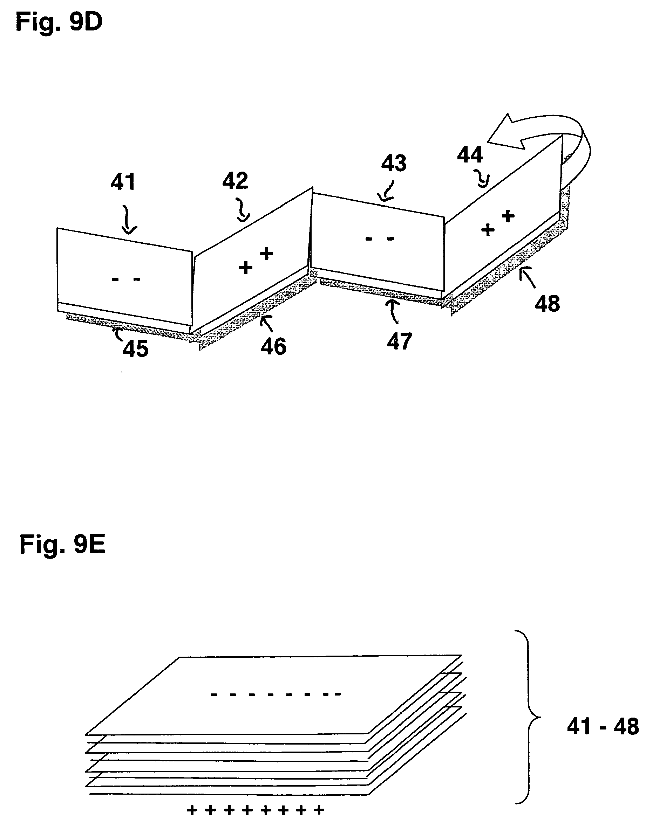

Die Figur 9D ist beispielhaft für den Ablauf weiterer Faltungsvorgänge entlang der Faltungslinien 3a skizziert. Durch die Übereinanderfaltung des bereits gefalteten Trägers 1 kommt es zu einer weiteren Überlagerung der Magnetfeldintensitäten, welche wegen der zunächst gegensätzlichen Polaritäten benachbarter Felder erhöhend wirkt.

Als Ergebnis ergibt sich die in Figur 9E dargestellte Anordnung, bei der es zu einer achtfachen Überhöhung der Magnetfeldintensität kommt, wiederum gleiche Ursprungsintensitäten angenommen.

Obwohl in dem dargestellten Ausführungsbeispiel mit einer Faltung entlang der Faltungslinie 3b begonnen wurde, ergibt auch jede andere Faltungsreihenfolge letztendlich das in Figur 9E dargestellte Ergebnis bezüglich der resultierenden Magnetfeldintensität.

In den zuvor beschriebenen Ausführungsbeipielen ist der Träger jeweils in rechteckiger Form beschrieben. Jedoch kann der Träger auch andere Formen wie z.B. eine runde Form aufweisen. Insbesondere kann der Träger auch kreisrund ausgeführt sein, wobei die Faltungslinien den Träger dann in einzelne sektorförmige Felder einteilen. Die Magnetfeldquellen benachbarter Sektoren wechseln sich hierbei vorzugsweise ab, so dass sich der zuvor beschriebene Effekt der erhöhenden Überlagerung ergibt.

## Patentansprüche

1. Magnetfeldapplikator, umfassend
einen flächigen Träger (1), der durch wenigstens zwei sich schneidende mechanisch definierte Faltungslinien (3a, 3b) in eine Anzahl Felder (41, ..., 48) geteilt und entlang der Faltungslinien (3a, 3b) faltbar ist, wobei wenigstens zwei Felder aus der Anzahl von Feldern (41, ..., 48) eine Magnetfeldquelle (21, ..., 28) aufweisen, die jeweils einen zur Erzeugung eines Magnetfelds geeigneten elektrischen Leiter umfasst und wobei die Magnetfeldquellen (21, ..., 28) derart in den Feldern (41, ..., 48) angeordnet sind, dass sich Magnetfeldintensitäten der Magnetfeldquellen (21, ..., 28) übereinander gefalteter Felder bei einer Faltung des Trägers (1) erhöhend überlagern.

2. Magnetfeldapplikator nach Anspruch 1,
bei dem die Faltungslinien (3a, 3b) durch Nähte (31) auf dem Träger (1) gebildet sind.

3. Magnetfeldapplikator nach Anspruch 1 oder 2,
bei dem benachbarte Felder durch Scharniere (32) oder Bänder miteinander verbunden sind, deren Achsen (33) die Faltungslinien (3a, 3b) definieren.

4. Magnetfeldapplikator nach einem der Ansprüche 1 bis 3,
bei dem benachbarte Felder Platten aufweisen, die entlang einer der Faltungslinien (3a, 3b) flexibel miteinander verbunden sind.

5. Magnetfeldapplikator nach einem der Ansprüche 1 bis 4,
bei dem die Faltungslinien (3a, 3b) sichtbar sind.

6. Magnetfeldapplikator nach einem der Ansprüche 1 bis 5,
bei dem die elektrischen Leiter als flache Spulen (210) ausgeführt sind.

7. Magnetfeldapplikator nach Anspruch 6,
bei dem die elektrischen Leiter mittels eines Druck- oder Ätzverfahrens auf einer Folie aufgebracht ist.

8. Magnetfeldapplikator nach Anspruch 6 oder 7,
bei dem zumindest einer der elektrischen Leiter über Nähte (6) in den Feldern (41, ..., 48) fixiert ist.

9. Magnetfeldapplikator nach einem der Ansprüche 6 bis 8,
bei dem die Spulen zwischen einen ersten und einen zweiten Anschluss (5) in Reihe und/oder parallel geschaltet sind.

10. Magnetfeldapplikator nach einem der Ansprüche 1 bis 9,
bei dem die elektrischen Leiter zusätzlich zur Wärmeerzeugung geeignet sind.

11. Magnetfeldapplikator nach einem der Ansprüche 1 bis 10,
bei dem wenigstens eine der Magnetfeldquellen (21, ..., 28) einen Permanentmagneten (215) aufweist.

12. Magnetfeldapplikator nach einem der Ansprüche 1 bis 11,
bei dem die Felder (41, ..., 48) im Wesentlichen die gleichen Abmessungen aufweisen.

13. Magnetfeldapplikator nach einem der Ansprüche 1 bis 12,
bei dem die Magnetfeldquellen (21, ..., 28) zur Erzeugung von im Wesentlichen gleichen Magnetfeldintensitäten eingerichtet sind.

14. Magnetfeldapplikator nach einem der Ansprüche 1 bis 13,
bei dem ein Material des Trägers (1) wenigstens eines der folgenden umfasst: einen textilen Stoff, ein Vlies, ein Gewebe, eine Membran, eine Kunststofffolie.

## Claims

1. A magnetic field applicator comprising
a sheet-like support (1) which is divided into a number of areas (41, ..., 48) by at least two intersecting, mechanically defined fold lines (3a, 3b) and can be folded along the fold lines (3a, 3b), at least two areas from the number of areas (41, ..., 48) comprising a magnetic field source (21, ..., 28) each including an electrical conductor which is suitable for generating a magnetic field, and said magnetic field sources (21, ..., 28) being arranged in the areas (41, ..., 48) in such a manner that the magnetic field intensities of the magnetic field sources (21, ..., 28) of areas folded to be on top of one another are superimposed so as to increase when folding the support (1).

2. The magnetic field applicator according to claim 1,
in which the fold lines (3a, 3b) are formed on the support (1) by seams (31).

3. The magnetic field applicator according to claim 1 or 2,
in which neighboring areas are interconnected by hinges (32) or ribbons whose axes (33) define the fold lines (3a, 3b).

4. The magnetic field applicator according to any of the claims 1 to 3,
in which neighboring areas comprise plates which are flexibly interconnected along one of the fold lines (3a, 3b).

5. The magnetic field applicator according to any of the claims 1 to 4,
in which the fold lines (3a, 3b) are visible.

6. The magnetic field applicator according to any of the claims 1 to 5,
in which the electrical conductors are implemented as flat coils (210).

7. The magnetic field applicator according to claim 6,
in which the electrical conductors are applied on a foil by means of a printing or etching process.

8. The magnetic field applicator according to claim 6 or 7,
in which at least one of the electrical conductors is fixed in the areas (41, ..., 48) by means of seams (6).

9. The magnetic field applicator according to any of the claims 6 to 8,
in which the coils are connected in series and/or parallel between a first and a second terminal (5).

10. The magnetic field applicator according to any of the claims 1 to 9,
in which the electrical conductors are additionally suitable for generating heat.

11. The magnetic field applicator according to any of the claims 1 to 10,
in which at least one of the magnetic field sources (21, ..., 28) comprises a permanent magnet (215).

12. The magnetic field applicator according to any of the claims 1 to 11,
in which the areas (41, ..., 48) substantially have the same dimensions.

13. The magnetic field applicator according to any of the claims 1 to 12,
in which the magnetic field sources (21, ..., 28) are adapted for generating substantially identical magnetic field intensities.

14. The magnetic field applicator according to any of the claims 1 to 13,
in which a material of the support (1) comprises at least one of the following: a textile cloth, a fleece, a fabric, a membrane, a plastic film.

## Revendications

1. Applicateur de champ magnétique, comprenant
un support plan (1), qui est divisé en un nombre de champs (41, ..., 48) par au moins deux lignes de pliage (3a, 3b) définies mécaniquement et se croisant, et qui est pliable le long des lignes de pliage (3a, 3b), sachant qu'au moins deux champs parmi le nombre de champs (41, ..., 48) présentent une source de champ magnétique (21, ..., 28) qui comprend respectivement un conducteur électrique apte à générer un champ magnétique et sachant que les sources de champ magnétique (21, ..., 28) sont disposées de telle façon dans les champs (41, ..., 48) que des intensités de champ magnétique des sources de champ magnétique (21, ..., 28) de champs pliés les uns aux dessus des autres se superposent de manière croissante lors d'un pliage du support (1).

2. Applicateur de champ magnétique selon la revendication 1,
dans lequel les lignes de pliage (3a, 3b) sont formées par des joints (31) sur le support (1).

3. Applicateur de champ magnétique selon la revendication 1 ou 2,
dans lequel des champs adjacents sont reliés entre eux par des charnières (32) ou des paumelles dont les axes (33) définissent les lignes de pliage (3a, 3b).

4. Applicateur de champ magnétique selon l'une des revendications 1 à 3,
dans lequel des champs adjacents présentent des plaques qui sont reliées entre elles de manière flexible le long d'une des lignes de pliage (3a, 3b).

5. Applicateur de champ magnétique selon l'une des revendications 1 à 4,
dans lequel les lignes de pliage (3a, 3b) sont visibles.

6. Applicateur de champ magnétique selon l'une des revendications 1 à 5,
dans lequel les conducteurs électriques sont exécutés comme bobines plates (210).

7. Applicateur de champ magnétique selon la revendication 6,
dans lequel les conducteurs électriques sont apposés sur une feuille moyennant un procédé d'impression ou de gravure.

8. Applicateur de champ magnétique selon la revendication 6 ou 7,
dans lequel au moins un des conducteurs électriques est fixé dans les champs (41, ..., 48) par des joints (6).

9. Applicateur de champ magnétique selon l'une des revendications 6 à 8,
dans lequel les bobines sont connectées en série et/ou en parallèle entre une première et une deuxième connexion (5).

10. Applicateur de champ magnétique selon l'une des revendications 1 à 9,
dans lequel les conducteurs électriques conviennent en outre pour la production de chaleur.

11. Applicateur de champ magnétique selon l'une des revendications 1 à 10,
dans lequel au moins une des sources de champ magnétique (21, ..., 28) présente un aimant permanent (215).

12. Applicateur de champ magnétique selon l'une des revendications 1 à 11,
dans lequel les champs (41, ..., 48) présentent sensiblement les mêmes dimensions.

13. Applicateur de champ magnétique selon l'une des revendications 1 à 12,
dans lequel les sources de champ magnétique (21, ..., 28) sont configurées pour générer des intensités de champ magnétique sensiblement égales.

14. Applicateur de champ magnétique selon l'une des revendications 1 à 13,
dans lequel un matériau du support (1) comprend au moins un des éléments suivants : une matière textile, une toile, un tissu, une membrane, une feuille en matière synthétique.
